# FASCICULE DE BREVET EUROPEEN

(11) **EP 2 033 938 B1**
(45) Date de publication et mention de la délivrance du brevet: **15.10.2014**
(21) Numéro de dépôt: 08290788.2
(22) Date de dépôt: 19.08.2008
(51) Int. Cl.: C01B 39/48, B01J 29/70, C10G 45/64, C07C 5/27

(54) **Préparation d'un matériau composite poreux à base de Zéolithe EU-1 et sa mise en oeuvre en isomérisation des aromatiques en C8**
Herstellung eines porösen Verbundmaterials auf der Grundlage von Zeolith EU-1 und dessen Verwendung bei der Isomerisierung von Aromaten in C8
Preparation of a porous composite material based on Zeolite EU-1 and its use for the isomerisation of aromatic hydrocarbons in C8

(30) Priorité: 04.09.2007 FR 0706233
(43) Date de publication de la demande: 11.03.2009
(73) Titulaire: IFP Energies nouvelles, 92852 Rueil-Malmaison Cedex (FR)
(72) Inventeur: Patarin, Joël, 68720 Flaxlanden (FR); Guillon, Emmanuelle, 69390 Vernaison (FR); Rouleau, Loic, 69390 Charly (FR); Goergen, Simone, 69007 Lyon (FR)

(56) Documents cités:
- WO-A-99/16709
- FR-A- 2 785 279
- ARNOLD A ET AL: "Dry-gel synthesis of zeolites [Al]EU-1 and [Ga]EU-1" MICROPOROUS AND MESOPOROUS MATERIALS, ELSEVIER SCIENCE PUBLISHING, NEW YORK, US, vol. 67, no. 2-3, 6 février 2004 (2004-02-06), pages 205-213, XP004485734 ISSN: 1387-1811
- ARNOLD A ET AL: "Insight into the dry-gel synthesis of gallium-rich zeolite [Ga]Beta" MICROPOROUS AND MESOPOROUS MATERIALS, ELSEVIER SCIENCE PUBLISHING, NEW YORK, US, vol. 62, no. 1-2, 14 août 2003 (2003-08-14), pages 97-106, XP004441138 ISSN: 1387-1811

## Description

### Domaine technique

La présente invention se rapporte au domaine de la préparation d'un matériau composite poreux comprenant des cristaux de zéolithe EU-1 à sa surface extérieure et à l'application dudit matériau pour l'isomérisation des composés aromatiques en C₈. Plus précisément, la préparation dudit matériau selon l'invention met en oeuvre la transformation en zéolithe EU-1 d'une structure amorphe et poreuse composée d'un ou plusieurs oxyde(s) inorganique(s). Cette transformation est effectuée lors d'un traitement hydrothermal réalisé en présence d'une quantité déterminée d'une phase aqueuse externe.

### Art antérieur

La zéolithe EU-1, de type structural EUO est décrite dans l'art antérieur (W.M. Meier et D.H. Olson, « Atlas of Zeolite Structure types », 5ème Edition, 2001) et présente un réseau microporeux monodimensionnel, dont le diamètre des pores est de 4,1 x 5,4 Å (1 Å = 1 Angstrôm = 1.10⁻¹⁰ m). N.A. Briscoe et al ont enseigné que ces canaux monodimensionnels possèdent des poches latérales de profondeur 8,1 Å et de diamètre 6,8 x 5,8 Å (Zeolites, 8, 74, 1988).

La demande de brevet européen EP-A-0.042.226 décrit la zéolithe EU-1 de formule suivante :
au moins 10 XO₂ : Y₂O₃ : 0,5-1,5 R_{2/n}O : 0-100 H₂O
où R représente un cation de valence n, X représente le silicium et/ou le germanium, Y représente au moins un élément choisi parmi l'aluminium, le fer, le gallium, le bore. Elle décrit également le procédé de préparation de ladite zéolithe EU-1, lequel comprend le mélange en milieu aqueux d'au moins une source d'un élément X, d'au moins une source d'un élément Y et d'un composé organique azoté jouant le rôle de structurant Q, qui est soit le dérivé alkylé d'un polyméthylène α-ω diammonium, soit un produit de dégradation dudit dérivé, soit encore des précurseurs dudit dérivé. Le mélange réactionnel est ensuite placé sous pression autogène, à une température comprise entre 85 et 250°C, jusqu'à la formation de cristaux de zéolithe. Le produit obtenu est récupéré par filtration, lavé, séché, calciné pour enlever le structurant organique et éventuellement soumis à des échanges ioniques pour obtenir la forme acide de la zéolithe EU-1. Il est connu de mettre en oeuvre la forme acide de la zéolithe EU-1 ainsi préparée dans un procédé d'isomérisation des composés aromatiques en C₈. La zéolithe EU-1, préparée par le procédé standard décrit dans la demande de brevet européen EP-A-0.042.226, est obtenue sous forme d'une poudre et nécessite une mise en forme avec un liant avant d'être utilisée comme catalyseur. Un métal hydrogénant est ensuite déposé sur la zéolithe ou/et sur le liant. Par exemple, le brevet US 6.057.486 décrit un catalyseur pour l'isomérisation des composés aromatiques en C₈ et ayant de 1 à 90% poids de zéolithe EU-1 mélangé d'une manière homogène avec un liant inorganique, par exemple de alumine, et ayant de 0,01 à 20% poids d'au moins un élément du groupe VIII de la classification périodique des éléments.

L'homogénéisation d'une poudre de zéolithe avec une matrice par les techniques de mise en forme est une opération délicate. Une alternative plus attractive pour former des catalyseurs est de déposer les cristaux de zéolithe sur un support directement lors de l'étape de synthèse de la zéolithe. Un autre avantage d'une telle procédure est que l'utilisation d'un liant pour fixer la zéolithe sur un support n'est plus nécessaire.
Van der Puil et al. (Microporous and Mesoporous Materials 27 (1999) 95-106), par exemple, ont immergé des supports en alumine alpha, dans une solution aqueuse renfermant tous les réactifs nécessaires à la formation d'une zéolithe. Ce mélange renfermant les supports inorganiques est soumis à un traitement hydrothermal pour permettre la cristallisation de la zéolithe. Les cristaux formés se déposent à la surface du support jusqu'à recouvrir complètement la surface externe de ce support.

D'autres procédés pour préparer des catalyseurs par voie directe lors de la synthèse de la zéolithe mettent en oeuvre la transformation en zéolithe de réactifs qui ont été mis en forme. La demande de brevet internationale WO 99/16709 décrit un procédé de préparation qui permet de conserver une certaine porosité intercristalline dans la structure zéolithique. Une structure inorganique réactive et poreuse, par exemple une structure silicique méso- et/ou macroporeuse, est utilisée comme réactif pour former la zéolithe. Cette structure est alors imprégnée à sec avec des solutions aqueuses des autres réactifs nécessaires à la formation d'une zéolithe (agent structurant, agent minéralisant, élément de charpente, etc.). Le traitement hydrothermal est alors effectué en absence d'une quantité d'eau suffisante, qui pourrait causer une gélification de surface substantielle. La cristallisation commence à la surface interne et progresse ensuite dans toute la structure. Au moins 15% de la structure inorganique de départ sont transformés en zéolithe. La morphologie macroscopique de la structure initiale est conservée et au moins 25% du volume poreux est conservé.

### Résumé et intérêt de l'invention

La présente invention porte sur un procédé de préparation d'un matériau composite poreux formé d'un coeur amorphe à base d'un mélange d'oxyde de silicium et d'oxyde d'aluminium sur lequel sont dispersés des cristaux de zéolithe EU-1, ledit procédé comportant au moins les étapes suivantes :
1) imprégnation d'un solide comprenant au moins un oxyde de silicium et au moins un oxyde d'aluminium avec au moins une solution aqueuse comprenant au moins un cation diammonium quaternaire Q de formule [R₁R₂R₃-N-(CH₂)ₙ-N-R₄R₅R₆]²⁺, n étant compris entre 3 et 12, R₁ à R₆, égaux ou différents, sont des groupement alkyles ou hydroxyalkyles avec 1 à 8 atomes de carbone, et jusqu'à 5 des groupements R₁ à R₆ peuvent être des atomes d'hydrogène, chaque étape d'imprégnation selon ladite étape 1) étant suivie d'un séchage à une température inférieure à 200°C,
2) le traitement hydrothermal, mis en oeuvre dans un autoclave de volume V (ml) sous vapeur d'eau et à une température T comprise entre 120 et 220°C, dudit solide issu de l'étape 1), la quantité d'eau préalablement introduite dans ledit autoclave étant strictement supérieure à une quantité volumique égale à V*[23,48*10⁻¹⁰*T³-48*10⁻⁸*T²+5*10⁻⁵*T-0,002] et inférieure ou égale 0,25*V, et est telle que ledit solide n'est pas en contact direct avec elle,
3) le séchage puis la calcination du solide issu de l'étape 2) de manière à obtenir ledit matériau composite poreux.

La préparation dudit matériau selon l'invention met en oeuvre la transformation en zéolithe EU-1 d'une structure amorphe et poreuse composée d'un ou plusieurs oxyde(s) inorganique(s). Cette transformation est effectuée lors dudit traitement hydrothermal réalisé en présence d'une quantité déterminée d'eau. Un phénomène de cristallisation se produit d'abord en surface externe du solide avant de progresser, en fonction du temps de synthèse, vers l'intérieur du solide. La synthèse est interrompue avant que le taux de conversion en zéolithe EU-1 dépasse 15% poids. Le coeur du matériau reste amorphe.

La présente invention porte également sur la préparation d'un catalyseur comprenant ledit matériau composite poreux, ledit catalyseur comprenant en outre au moins un métal du groupe VIII de la classification périodique des éléments. Conformément à l'invention, les cristaux de zéolithe EU-1 se trouvent dispersés sur la surface externe dudit coeur dudit matériau composite poreux et il n'est pas nécessaire d'utiliser un liant pour la mise en forme de la zéolithe ce qui constitue un précieux avantage pour la préparation du catalyseur selon l'invention. Ledit catalyseur est avantageusement utilisé dans un procédé d'isomérisation d'une coupe aromatique comprenant au moins un composé aromatique à huit atomes de carbone par molécule. Il a été découvert, de façon surprenante, qu'un tel catalyseur, par exemple sous forme de billes ou d'extrudés, comprenant un matériau composite poreux formé d'un coeur amorphe sur lequel sont dispersés des cristaux de zéolithe EU-1, conduit à des performances catalytiques améliorées en terme de rendement en xylènes lorsque ledit catalyseur est utilisé dans un procédé d'isomérisation d'une coupe aromatique comprenant au moins un composé aromatique à huit atomes de carbone par molécule.

### Description de l'invention

La présente invention a pour objet un procédé de préparation d'un matériau composite poreux formé d'un coeur amorphe à base d'un mélange d'oxyde de silicium et d'oxyde d'aluminium sur lequel sont dispersés des cristaux de zéolithe EU-1, ledit procédé comportant au moins les étapes suivantes :
1) imprégnation d'un solide comprenant au moins un oxyde de silicium et au moins un oxyde d'aluminium avec au moins une solution aqueuse comprenant au moins un cation diammonium quaternaire Q de formule [R₁R₂R₃-N-(CH₂)ₙ-N-R₄R₅R₆]^{2+,} n étant compris entre 3 et 12, R₁ à R₆, égaux ou différents, sont des groupements alkyles ou hydroxyalkyles avec 1 à 8 atomes de carbone, et jusqu'à 5 des groupements R₁ à R₆ peuvent être des atomes d'hydrogène, chaque étape d'imprégnation selon ladite étape 1) étant suivie d'un séchage à une température inférieure à 200°C,
2) le traitement hydrothermal, mis en oeuvre dans un autoclave de volume V (ml) sous vapeur d'eau et à une température T comprise entre 120 et 220°C, dudit solide issu de l'étape 1), la quantité d'eau préalablement introduite dans ledit autoclave étant strictement supérieure à une quantité volumique égale à V*[23,48*10⁻¹⁰*T³-48*10⁻⁸*T²+5*10⁻⁵*T-0,002] et inférieure ou égale à 0,25*V, et est telle que ledit solide n'est pas en contact direct avec elle,
3) le séchage puis la calcination du solide issu de l'étape 2) de manière à obtenir ledit matériau composite poreux.

Conformément à l'invention, le matériau préparé par le procédé de l'invention est un matériau composite poreux formé d'un coeur amorphe à base d'un mélange d'oxyde de silicium et d'oxyde d'aluminium sur lequel sont dispersés des cristaux de zéolithe EU-1. Ledit matériau ainsi obtenu est majoritairement macroporeux. Il a des pores plus larges et un volume poreux d'au moins 30% inférieur au volume poreux du solide comprenant au moins un oxyde de silicium et au moins un oxyde d'aluminium utilisé pour la mise en oeuvre de ladite étape 1) du procédé de l'invention. Le coeur amorphe dudit matériau est formé d'un mélange d'oxyde de silicium et d'oxyde d'aluminium. Des cristaux et des agglomérats de cristaux de zéolithe EU-1 sont dispersés sur la surface externe dudit coeur sans qu'une couche continue et cristallisée de zéolithe EU-1 ne recouvre entièrement ladite surface. Ledit matériau est caractérisé par microscopie électronique en transmission (MET) et par microscopie électronique à balayage (MEB). Ledit matériau composite poreux préparé selon le procédé de l'invention peut se présenter sous différentes formes macroscopiques, par exemple sous forme d'extrudés ou de billes, de préférence sous forme d'extrudés. Conformément à l'invention, ledit matériau composite poreux se présente sous la même forme macroscopique que celle du solide comprenant au moins un oxyde de silicium et au moins un oxyde d'aluminium utilisé pour la mise en oeuvre de ladite étape 1) du procédé de l'invention.

Le solide comprenant au moins un oxyde de silicium et au moins un oxyde d'aluminium utilisé pour la mise en oeuvre de l'étape 1) du procédé de préparation du matériau selon l'invention est par exemple une silice-alumine amorphe présentant un rapport molaire global SiO₂/Al₂O₃ d'au moins 10 et de préférence compris entre 10 et 150. Ladite silice-alumine peut se présenter sous différentes formes macroscopiques, par exemple des extrudés, des billes ou toute autre forme connue de l'Homme du métier. La taille moyenne de ladite silice-alumine peut varier entre 0,1 et 5 mm et de manière préférée entre 0,5 et 3 mm. Elle possède un système poreux ordonné ou désordonné, composé de méso- et /ou macropores, c'est-à-dire avec un diamètre moyen des pores supérieur à 2 nm et un volume poreux compris entre 0,01 et 2 ml.g⁻¹ et de préférence entre 0,1 et 1,5 ml.g⁻¹. Conformément à l'invention, ledit matériau composite poreux se présente sous la même forme macroscopique que celle de la silice-alumine.

Le solide comprenant au moins un oxyde de silicium et au moins un oxyde d'aluminium utilisé pour la mise en oeuvre de l'étape 1) du procédé de préparation du matériau selon l'invention peut également être obtenu par imprégnation d'une solution aqueuse contenant de l'aluminium sur une structure constituée d'au moins oxyde de silicium. L'oxyde de silicium utilisé est une structure pouvant se présenter sous différentes formes macroscopiques, par exemple des extrudés, des billes ou toute autre forme connue de l'Homme du métier. Le diamètre moyen de ladite structure constituée d'oxyde de silicium utilisée peut varier entre 0,1 et 5 mm et de manière préférée entre 0,5 et 3 mm. Ladite structure constituée d'oxyde de silicium possède un système poreux ordonné ou désordonné, composé de méso- et /ou macropores, c'est-à-dire avec un diamètre moyen des pores supérieur à 2 nm et un volume poreux compris entre 0,01 et 2 ml.g⁻¹ et de préférence entre 0,1 et 1,5 ml.g⁻¹.

Selon le mode de réalisation d'imprégnation de ladite solution aqueuse contenant de l'aluminium sur ladite structure d'oxyde de silicium, ledit solide comprenant au moins un oxyde de silicium et au moins un oxyde d'aluminium présente soit de l'aluminium réparti de façon homogène au sein de la structure constituée d'oxyde de silicium soit de l'aluminium déposé sur la surface externe de ladite structure constituée d'oxyde de silicium.
Pour obtenir un solide comprenant au moins un oxyde de silicium et au moins un oxyde d'aluminium présentant de l'aluminium réparti de façon homogène au sein de la structure constituée d'oxyde de silicium, la structure constituée d'oxyde de silicium est imprégnée à température ambiante, à sec, en excès ou selon tout autre méthode connue de l'Homme du métier, avec une solution aqueuse comportant une source d'aluminium. Le mode d'imprégnation préféré est l'imprégnation à sec et le volume de la solution aqueuse contenant de l'aluminium à imprégner est de préférence égal au volume poreux de la structure constituée d'oxyde de silicum.
Après imprégnation, ledit solide comprenant au moins un oxyde de silicium et au moins un oxyde d'aluminium présentant de l'aluminium réparti de façon homogène au sein de la structure constituée d'oxyde de silicium présente un rapport molaire global SiO₂/A1₂O₃ d'au moins 10 et de préférence compris entre 10 et 150.
Pour obtenir un solide comprenant au moins un oxyde de silicium et au moins un oxyde d'aluminium présentant de l'aluminium déposé sur la surface externe de ladite structure constituée d'oxyde de silicium, la structure constituée d'oxyde de silicium est d'abord imprégnée avec un liquide dépourvu d'aluminium. De préférence, on réalise une imprégnation à sec, à température ambiante, avec un volume de liquide égal ou légèrement inférieur au volume poreux de la structure d'oxyde de silicium. Le volume poreux de ladite structure constituée d'oxyde de silicium est rempli au moins à 90%, de manière préférée entre 95 et 99% avec ce liquide. Le liquide dépourvu d'aluminium et utilisé pour boucher la porosité de la structure constituée d'oxyde de silicium est avantageusement choisi parmi de l'eau, un solvant organique et un mélange d'eau et de solvant organique. On peut employer un alcool, un composé comportant une fonction ammonium quaternaire, un acide organique, un polyol en tant que solvant organique ou d'autres solvants connus de l'Homme du Métier, ledit solvant organique ayant la propriété de se décomposer entre 100 et 1000°C pour être éliminé par calcination. Ensuite ladite structure est imprégnée, par une imprégnation à sec ou en excès, de préférence par une imprégnation à sec, avec une solution aqueuse d'une source d'aluminium. De cette manière, la diffusion d'aluminium dans les pores de la structure constituée d'oxyde de silicium est réduite voire empêchée et l'aluminium est déposé majoritairement sur la surface externe de ladite structure. Le solide comprenant au moins un oxyde de silicium et au moins un oxyde d'aluminium utilisé pour la mise en oeuvre de ladite étape 1) du procédé de préparation selon l'invention présente un rapport molaire global SiO₂/Al₂O₃ d'au moins 200. Le matériau composite poreux préparé selon le procédé de l'invention et préparé à partir d'un solide présentant de l'aluminium déposé sur la surface externe de ladite structure constituée d'oxyde de silicium est caractérisé par un coefficient de répartition de l'aluminium inférieur à 0,5, de préférence inférieur à 0,4 et de manière très préférée inférieur à 0,3. Ledit coefficient de répartition de l'aluminium, obtenu par microsonde de Castaing, correspond au rapport des concentrations de l'aluminium au coeur d'un extrudé ou d'une bille dudit matériau composite poreux par rapport au bord de ce même extrudé ou de cette bille selon la forme macroscopique dudit matériau.

Conformément à l'invention, ledit matériau composite poreux se présente sous la même forme macroscopique que celle de la structure constituée d'oxyde de silicium lorsque ledit solide utilisée pour la mise en oeuvre de l'étape 1) est obtenu par imprégnation d'une solution aqueuse contenant de l'aluminium sur ladite structure d'oxyde de silicium.

La source d'aluminium employée pour l'imprégnation sur la structure constituée d'oxyde de silicium peut avantageusement être tout composé comprenant l'aluminium et pouvant libérer l'aluminium en solution aqueuse sous forme réactive. La source d'aluminium est de préférence de l'aluminate de sodium ou un sel d'aluminium, par exemple du chlorure, du nitrate, de l'hydroxyde ou du sulfate, ou encore un alkoxyde d'aluminium.

Après l'imprégnation de l'aluminium, lequel est soit réparti de façon homogène au sein de la structure constituée d'oxyde de silicium soit déposé sur la surface externe de ladite structure constituée d'oxyde de silicium, ledit solide comprenant de l'oxyde de silicium et de l'oxyde d'aluminium est avantageusement séché à une température inférieure à 200°C et de préférence inférieure à 120°C et de manière très préférée inférieure à 100°C et/ou avantageusement calciné sous air à une température comprise entre 150°C et 1000°C, de manière préférée entre 300°C et 700°C et de manière encore plus préférée entre 400°C et 650°C pendant une durée de 1 heure à 20 heures.
Conformément à l'étape 1) du procédé de préparation selon l'invention, ledit solide comprenant au moins un oxyde de silicium et au moins un oxyde d'aluminium est imprégné avec au moins une solution aqueuse comprenant au moins un cation diammonium quaternaire Q de formule [R₁R₂R₃-N-(CH₂)ₙ-N-R₄R₅R₆]²⁺, n étant compris entre 3 et 12, R₁ à R₆, égaux ou différents, sont des groupements alkyles ou hydroxyalkyles avec 1 à 8 atomes de carbone, et jusqu'à 5 des groupements R₁ à R₆ peuvent être des atomes d'hydrogène. De manière préférée, n est égal à 6, R₁, R₂, R₃, R₄, R₅ et R₆ sont des groupes méthyles : il s'agit du cation hexaméthonium. L'imprégnation dudit solide renfermant des oxydes de silicium et d'aluminium est réalisée à température ambiante, à sec, en excès ou selon tout autre méthode connue de l'Homme du métier, avec une solution aqueuse comprenant au moins ledit cation diammonium quaternaire Q. Le mode d'imprégnation préféré est l'imprégnation à sec et le volume de la solution aqueuse comprenant au moins ledit cation Q à imprégner est inférieur ou égal au volume poreux du solide comprenant au moins un oxyde de silicium et au moins un oxyde d'aluminium. Ledit cation Q est présent sous forme hydroxyde ou comme sel halogéné, de préférence sous forme hydroxyde. De manière très préférée, ladite solution aqueuse comprenant ledit cation Q comprend de l'hydroxyde d'hexaméthonium.
L'imprégnation de la solution aqueuse comprenant ledit cation diammonium quaternaire Q est avantageusement répétée plusieurs fois, par exemple trois imprégnations sont réalisées. Après chaque étape d'imprégnation on effectue un séchage à une température inférieure à 200°C et de préférence inférieure à 120°C et de manière très préférée inférieure à 100°C pour évaporer l'eau et libérer le volume poreux pour une imprégnation successive.

A l'issue de(s) étape(s) d'imprégnation et de séchage selon ladite étape 1) du procédé de l'invention, le solide présente la composition molaire suivante :

| | |
|---|---|
| SiO₂/Al₂O₃ | : au moins 10, |
| OH⁻/SiO₂ | : 0,1 à 6,0, de préférence de 0,1 à 1,0, |
| (M + Q)/Al₂O₃ | : 0,5 à 100, |
| Q/(M + Q) | : 0,1 à 1, |
| H₂O/SiO₂ | : 0 à 20, |

M représente un cation monovalent, provenant des sources de silicium et d'aluminium, choisi parmi les métaux alcalins et l'ammonium. De manière préférée, M est le sodium.

Conformément à l'étape 2) du procédé de préparation selon l'invention, ledit solide issu de ladite étape 1) et renfermant au moins les oxydes de silicium et d'aluminium est soumis à un traitement hydrothermal, mis en oeuvre dans un autoclave de volume V (ml) sous vapeur d'eau et à une température T comprise entre 120 et 220°C, de manière à conduire à la formation de cristaux de zéolithe EU-1, dispersés à la surface dudit solide. Selon ladite étape 2), la quantité d'eau préalablement introduite dans ledit autoclave est strictement supérieure à un volume égal à V*[23,48*10⁻¹⁰*T³-48*10⁻⁸*T²+5*10⁻⁵*T-0,002] et est inférieure ou égale à un volume égal à 0,25*V, et est telle que ledit solide n'est pas en contact direct avec l'eau.

Pour la mise en oeuvre de ladite étape 2) du procédé de l'invention, ledit solide issu de l'étape 1) du procédé de l'invention est avantageusement placé sur un support inoxydable, localisé dans la partie supérieure ou centrale d'un autoclave. Au fond de cet autoclave est présente de l'eau qui ne se trouve pas en contact direct avec ledit solide. La quantité d'eau introduite au fond de l'autoclave est choisie de manière à ce qu'elle soit suffisante pour que l'atmosphère autour dudit solide soit saturée en vapeur d'eau et que l'eau re-condense sur ledit solide. Le volume d'eau introduit au fond de l'autoclave est strictement supérieur à un volume égal à V*[23,48*10⁻¹⁰*T³-48*10⁻⁸*T²+5*10⁻⁵*T-0,002] et est inférieur ou égal à un volume égal à 0,25*V, V étant le volume de l'autoclave exprimé en ml et T la température du traitement hydrothermal exprimée en °C. De manière préférée, le traitement hydrothermal est réalisé à une température de 180°C dans un autoclave de 100 ml, la quantité d'eau introduite dans le fond de l'autoclave et ne se trouvant pas en contact direct avec le solide est supérieure à 0,5 ml, de préférence au moins égale à 1 ml et de manière encore plus préférée au moins égale à 4 ml. L'eau qui re-condense sur le solide provoque une gélification de la surface externe dudit solide, de sorte que se produit un phénomène de cristallisation qui commence à la surface externe dudit solide avant de progresser, en fonction du temps de synthèse, vers l'intérieur du solide. La " gélification de surface" correspond, au sens de la présente invention, à une dissolution importante, en surface, du solide inorganique accompagnée d'une perte de porosité en surface et de la formation, également en surface, d'une phase amorphe plus dense. Le coeur est moins affecté par le phénomène de gélification de surface de sorte que sa porosité est moins modifiée que celle en surface au cours de la cristallisation. Conformément à l'invention, le phénomène de gélification de la surface externe se produit d'une manière telle que le coeur du matériau composite poreux préparé selon le procédé de l'invention reste amorphe, la gélification de la surface externe générant une barrière diffusionnelle qui limite la progression de la cristallisation de la surface externe vers le coeur. Aucun phénomène de cristallisation au coeur dudit matériau n'est observé, les cristaux de zéolithe EU-1 étant uniquement présents sur la surface externe dudit coeur amorphe.

Le traitement hydrothermal, effectué pour la mise en oeuvre de ladite étape 2) du procédé de l'invention, est réalisé sous une pression de réaction autogène, à une température comprise entre 120°C et 220°C, de préférence entre 140°C et 200°C, et de manière très préférée à une température comprise entre 150 et 190°C. La durée de synthèse est de 1 heure à plusieurs jours, de manière préférée, de 6 heures à 7 jours et de manière encore plus préférée de 12 heures à 4 jours. La synthèse est avantageusement arrêtée lorsque le taux de conversion en zéolithe EU-1 est inférieur ou égal à 15% poids, de manière préférée inférieur ou égal à 10% poids. Le taux de conversion est d'au moins 1% poids, de préférence d'au moins 4% poids, en surface et est inférieur à 3% poids et de manière préférée inférieur à 1% poids au coeur du matériau.
Le taux de conversion en zéolithe EU-1 est déterminé par diffraction de rayons X par rapport à un échantillon de référence entièrement constitué de zéolithe EU-1 100% cristallin. Plus précisément, il est calculé à partir du diagramme de diffraction du matériau composite poreux préparé selon le procédé de l'invention par comparaison avec celui dudit échantillon de référence entièrement constitué de zéolithe EU-1 100% cristallin. Ledit échantillon de référence est constitué d'une zéolithe EU-1 préparée selon l'enseignement décrit dans la demande de brevet EP A-0.042.226 et qui est 100% cristallin. Le taux de conversion, exprimé en % poids, correspond au rapport de la surface des pics du matériau composite poreux préparé selon le procédé de l'invention sur la surface des pics de la zéolithe EU-1 de référence 100% cristallisée, après soustraction du bruit de fond dans le domaine d'angle de diffraction 2θ de 13° à 32°.

A l'issue de ladite étape 2) du procédé de préparation selon l'invention, le matériau obtenu se trouve sous sa forme brute de synthèse. Il est récupéré, lavé puis conformément à l'étape 3) du procédé de préparation selon l'invention, il est séché, préférentiellement à une température inférieure à 200°C et de manière très préférée inférieure à 120°C puis il est calciné. La calcination est réalisée par les méthodes connues de l'Homme du Métier, par exemple sous un flux d'air sec pour éliminer le cation diammonium quaternaire Q jouant le rôle de structurant organique occlus dans les micropores du matériau. La calcination est avantageusement effectuée à une température comprise entre 150°C et 1000°C, de manière plus avantageuse comprise entre 300°C et 700°C et de manière encore plus avantageuse comprise entre 400°C et 650°C pendant une durée préférentiellement comprise entre 1 heure et 40 heures et de manière plus préférée comprise entre 10 et 20 heures. Si le matériau calciné renferme des cations M⁺ sous forme de cations de métaux alcalins, on effectue au moins une étape d'échange ionique, par exemple avec au moins une solution aqueuse de NH₄NO₃, pour éliminer au moins une partie et de préférence tous les cations de métaux alcalins.

La présente invention a également pour objet la préparation d'un catalyseur à partir du matériau composite poreux préparé conformément au procédé de l'invention décrit ci-dessus. Ledit procédé de préparation dudit catalyseur comprend au moins une étape consistant à imprégner ledit matériau composite poreux avec au moins un métal du groupe VIII de la classification périodique des éléments et éventuellement au moins un métal choisi parmi les métaux des groupes IIIA et IVA.
La préparation du catalyseur selon l'invention, après la préparation du matériau conformément aux trois étapes de préparation du procédé décrit ci-dessus, peut être effectuée par toute méthode connue de l'Homme du métier. De manière préférée, à la suite de la calcination réalisée à la fin de l'étape 3) du procédé de préparation du matériau selon l'invention et des éventuelles étapes d'échange ionique, par exemple avec une solution aqueuse de NH₄NO₃, au moins un métal du groupe VIII est introduit sur ledit matériau, à savoir soit majoritairement sur le coeur, soit majoritairement sur les cristaux de zéolithe EU-1 soit encore sur l'ensemble zéolithe - coeur. Le dépôt dudit métal sur le coeur est avantageusement effectué par la technique d'imprégnation à sec, la technique d'imprégnation en excès ou par échange ionique. Lorsque plusieurs métaux sont introduits, ceux-ci peuvent être introduits soit tous de la même façon soit par des techniques différentes.
Tous les précurseurs de métaux du groupe VIII conviennent pour le dépôt d'un ou de plusieurs métal(ux) du groupe VIII sur le matériau préparé selon le procédé de l'invention en trois étapes décrit ci-dessus. En particulier, pour tout métal noble du groupe VIII, on peut utiliser des composés ammoniaqués ou des composés tels que par exemple le chloroplatinate d'ammonium, le dichlorure de platine dicarbonyle, l'acide hexahydroxyplatinique, le chlorure de palladium ou le nitrate de palladium. Le platine est généralement introduit sous forme d'acide hexachloroplatinique. L'introduction du métal noble du groupe VIII est de préférence effectuée par imprégnation à l'aide d'une solution aqueuse ou organique de l'un des composés métalliques cités ci-dessus. Parmi les solvants organiques utilisables, on peut citer les hydrocarbures paraffiniques, naphténiques ou aromatiques contenant par exemple de 6 à 12 atomes de carbone par molécule, et les composés organiques halogénés contenant par exemple de 1 à 12 atomes de carbone par molécule. On peut citer par exemple le n-heptane, le méthylcyclohexane, le toluène et le chloroforme. On peut aussi utiliser les mélanges de solvants.
Le contrôle de certains paramètres mis en oeuvre lors du dépôt, en particulier la nature du précurseur du (des) métal(ux) du groupe VIII utilisé(s), permet d'orienter le dépôt du(es)dit(s) métal(ux) majoritairement sur le coeur du matériau préparé selon le procédé de l'invention ou sur les cristaux de zéolithe.
Ainsi, pour introduire le(s) métal(ux) du groupe VIII, préférentiellement le platine et/ou le palladium, majoritairement sur le coeur, on peut mettre en oeuvre un échange anionique avec de l'acide hexachloroplatinique et/ou de l'acide hexachloropalladique, en présence d'un agent compétiteur, par exemple de l'acide chlorhydrique, le dépôt étant en général suivi d'une calcination, par exemple à une température comprise entre 350 et 550°C et pendant une durée comprise entre 1 et 4 heures. Avec de tels précurseurs, le(s) métal(ux) du groupe VIII est(sont) déposé(s) majoritairement sur le coeur et le(s)dit(s) métal(ux) présente(nt) une bonne dispersion et une bonne répartition macroscopique à travers les extrudés ou les billes du catalyseur.
On peut aussi envisager de déposer le(s) métal(ux) du groupe VIII, préférentiellement le platine et/ou le palladium, par échange cationique de manière à ce que le(s)dit(s) métal(ux) soi(en)t déposé(s) majoritairement sur les cristaux de zéolithe. Ainsi, dans le cas du platine, le précurseur peut être par exemple choisi parmi :
- les composés ammoniaqués tels que les sels de platine (II) tétrammines de formule Pt(NH₃)₄X₂, les sels de platine (IV) hexammines de formule Pt(NH₃)₆X₄; les sels de platine (IV) halogénopentammines de formule (PtX(NH₃)₅)X₃; les sels de platine N-tétrahalogénodiammines de formule PtX₄(NH₃)₂; et
- les composés halogénés de formule H(Pt(acac)₂X);
X étant un halogène choisi dans le groupe formé par le chlore, le fluor, le brome et l'iode, X étant de préférence le chlore, et "acac" représentant le groupe acétylacétonate (de formule brute C₅H₇O₂), dérivé de l'acétylacétone. Avec de tels précurseurs, le(s) métal(ux) du groupe VIII est(sont) déposé(s) majoritairement sur les cristaux de zéolithe et le(s)dit(s) métal(ux) présente(nt) une bonne dispersion et une bonne répartition macroscopique à travers les extrudés ou les billes de catalyseur.
L'imprégnation à sec du métal du groupe VIII sur le matériau conduit au dépôt dudit métal à la fois sur le coeur et sur les cristaux de zéolithe.
De préférence, on utilisera la technique d'imprégnation à sec, pour favoriser le dépôt du métal du groupe VIII en croûte du matériau composite poreux préparé selon le procédé de l'invention en trois étapes décrit ci-dessus: le coefficient de répartition macroscopique du(des)dit(s) métal(ux) du groupe VIII, calculé à partir du profil de répartition du(des)dit(s) métal(ux) du groupe VIII déterminé par microsonde de Castaing, est inférieur à 0,7, de préférence inférieur à 0,6. Ledit coefficient est défini comme le rapport des concentrations du(des)dit(s) métal(ux) du groupe VIII au coeur d'un extrudé ou d'une bille du catalyseur par rapport au bord de ce même extrudé ou de cette même bille selon la forme macroscopique dudit catalyseur.

Dans le cas où le catalyseur préparé selon l'invention contient également au moins un métal choisi parmi les métaux des groupes IIIA et IVA, toutes les techniques de dépôt d'un tel métal connues de l'homme du métier et tous les précurseurs de tels métaux peuvent convenir.
On peut ajouter le(s) métal(ux) du groupe VIII et celui(ceux) des groupes IIIA et IVA, soit séparément soit simultanément dans au moins une étape unitaire. Lorsqu'au moins un métal des groupes IIIA et IVA est ajouté séparément, il est préférable qu'il soit ajouté après le métal du groupe VIII.
Le métal additionnel choisi parmi les métaux des groupes IIIA et IVA peut être introduit par l'intermédiaire de composés tels que par exemple les chlorures, les bromures et les nitrates des métaux des groupes IIIA et IVA. Par exemple dans le cas de l'indium, on utilise avantageusement le nitrate ou le chlorure. Le métal additionnel choisi parmi les métaux des groupes IIIA et IVA peut également être introduit sous la forme d'au moins un composé organique choisi dans le groupe constitué par les complexes dudit métal, en particulier les complexes polycétoniques du métal et les hydrocarbylmétaux tels que les alkyles, les cycloalkyles, les aryles, les alkylaryles et les arylalkyles métaux. Dans ce dernier cas, l'introduction du métal est avantageusement effectuée à l'aide d'une solution du composé organométallique dudit métal dans un solvant organique. On peut également employer des composés organohalogénés du métal. Comme composés organiques de métaux, on peut citer en particulier le tétrabutylétain, dans le cas de l'étain, et le triphénylindium, dans le cas de l'indium.
Si le métal additionnel choisi parmi les métaux des groupes IIIA et IVA est introduit avant le métal du groupe VIII, le composé du métal IIIA ou IVA utilisé est généralement choisi dans le groupe constitué par l'halogénure, le nitrate, l'acétate, le tartrate, le carbonate et l'oxalate du métal. L'introduction est alors avantageusement effectuée en solution aqueuse. Mais il peut également être introduit à l'aide d'une solution d'un composé organométallique du métal, par exemple le tétrabutylétain. Dans ce cas, avant de procéder à l'introduction d'au moins un métal du groupe VIII, on procédera à une calcination sous air.
De plus, des traitements intermédiaires tels que par exemple une calcination et/ou une réduction peuvent être appliqués entre les dépôts successifs des différents métaux.
La préparation du catalyseur se termine généralement par une calcination, habituellement à une température comprise entre 250°C et 600°C, pour une durée comprise entre 0,5 et 10 heures, de préférence précédée d'un séchage, par exemple à l'étuve, à une température allant de la température ambiante à 250°C, de préférence de 40°C à 200°C. Ladite étape de séchage est de préférence menée pendant la montée en température nécessaire pour effectuer ladite calcination. A la suite de la calcination, on procède éventuellement à une réduction sous hydrogène, généralement à une température comprise entre 300 et 600°C, de préférence entre 350°C et 550°C, et pour une durée comprise entre 1 et 10 heure(s), de préférence entre 2 et 5 heures, de façon à obtenir le(les)dit(s) métal(ux) principalement sous forme réduite nécessaire à l'activité catalytique.
Dans le cas où le catalyseur préparé selon la présente invention contient du soufre, le soufre est introduit sur le catalyseur calciné, contenant le ou les élément(s) cité(s) précédemment, soit *in situ* avant la réaction catalytique, soit *ex situ.* La sulfuration s'effectue en utilisant tout agent sulfurant bien connu de l'Homme du métier, tel que par exemple le disulfure de diméthyle ou le sulfure d'hydrogène. La sulfuration éventuelle intervient après la réduction. Dans le cas d'une sulfuration *in situ,* la réduction, si le catalyseur n'a pas été préalablement réduit, intervient avant la sulfuration. Dans le cas d'une sulfuration *ex situ,* on effectue la réduction puis la sulfuration.

Le catalyseur ainsi préparé contient, outre un coeur amorphe sur lequel sont dispersés des cristaux de zéolithe EU-1, au moins un métal du groupe VIII de la classification périodique des éléments, de préférence choisi dans le groupe formé par le platine et le palladium, de manière encore plus préférée le platine. La teneur pondérale du(des)dit(s) métal(ux) du groupe VIII est généralement comprise entre 0,01 et 2,0%, de préférence entre 0,05 et 1,0%. Ledit catalyseur comprend éventuellement au moins un métal additionnel choisi parmi les métaux des groupes IIIA et IVA de la classification périodique des éléments, de préférence choisi parmi l'indium et l'étain. La teneur pondérale du(des)dit(s) métal(ux) additionnel(s) est généralement comprise entre 0,01 et 2,0%, de préférence entre 0,05 et 1,0%. Ledit catalyseur comprend également avantageusement du soufre, dont la teneur est telle que le rapport du nombre d'atomes de soufre sur le nombre d'atomes de métal du groupe VIII déposés est compris entre 0,5 et 2.

La présente invention a également pour objet un procédé d'isomérisation d'une coupe contenant au moins un composé aromatique à huit atomes de carbone par molécule, ledit procédé comprenant la mise en contact de ladite coupe aromatique avec au moins ledit catalyseur préparé selon ledit procédé décrit ci-dessus et présent dans un réacteur catalytique. Ladite coupe aromatique contenant au moins un composé aromatique ayant huit atomes de carbone par molécule comprend en particulier comme composé aromatique ayant huit atomes de carbone par molécule soit uniquement un mélange de xylènes, soit uniquement de l'éthylbenzène soit un mélange de xylène(s) et d'éthylbenzène.

Le catalyseur utilisé dans le procédé d'isomérisation des coupes C₈ aromatiques selon la présente invention est sous forme de billes ou d'extrudés.

Ledit procédé d'isomérisation est mis en oeuvre généralement selon les conditions opératoires suivantes :
- une température comprise entre 300°C et 500°C, de préférence entre 320°C et 450°C et de manière encore plus préférée entre 340°C et 430°C,
- une pression partielle d'hydrogène comprise entre 0,3 et 1,5 MPa, de préférence entre 0,4 et 1,2 MPa et de manière encore préférée entre 0,7 et 1,2 MPa,
- une pression totale comprise entre 0,45 et 1,9 MPa, de préférence entre 0,6 et 1,5 MPa,
- une vitesse spatiale d'alimentation, exprimée en kilogramme de charge introduite par kilogramme de catalyseur et par heure, comprise entre 0,25 et 30h⁻¹, de préférence entre 1 et 10h⁻¹, et de manière encore préférée entre 2 et 6 h⁻¹.

Les exemples qui suivent illustrent l'invention sans pour autant en limiter la portée. Les matériaux préparés dans les exemples 1 à 6 suivant sont caractérisés par diffraction des rayons X (Panalytical, X'Pert), par microscopie électronique à balayage (JEOL, JSM 6340), par microscopie électronique à transmission (FEI, Tecnai) et par microsonde de Castaing (JEOL, 8800R).

L'échantillon de référence entièrement constitué de zéolithe EU-1, utilisé dans les exemples qui suivent, est préparé selon l'enseignement décrit dans la demande de brevet EP A-0.042.226 et est 100% cristallin.

### Exemple 1 (invention) : Préparation d'un matériau composite poreux M1 renfermant 5% poids de zéolithe EU-1.

Comme structure poreuse et amorphe, on prend 7 g d'extrudés de silice fournie par la société Saint Gobain NorPro, lesquels présentent un diamètre moyen de 1,5 mm, un volume poreux de 1 mL.g⁻¹ et un diamètre moyen de pores de 10 nm.
Ces extrudés de silice sont alors imprégnés à sec et à température ambiante avec une solution comprenant 0,55 g d'aluminate de sodium (Carlo Erba) dans 7 mL d'eau distillée. Les extrudés imprégnés sont ensuite calcinés sous air à 500°C pendant 2 heures.
Une solution aqueuse d'hydroxyde d'hexaméthonium est préparée, en laissant réagir 6 g de bromure d'hexaméthonium (Acros) avec 4,6 g d'oxyde d'argent (Alfa Aesar) dans 12 g d'eau distillée. Ce mélange est laissé sous agitation pendant une nuit à l'abri de la lumière. Après séparation du précipité d'AgBr par filtration, on récupère une solution à 25% massique en hydroxyde d'hexaméthonium.

Les extrudés sont ensuite imprégnés à sec et à température ambiante avec 11 g de cette solution d'hydroxyde d'hexaméthonium. Cette imprégnation est réalisée en 3 étapes. Après chaque imprégnation, les extrudés sont séchés à 80°C, pour évaporer l'eau et libérer du volume poreux.
A l'issue de ces étapes d'imprégnation et de séchage, les extrudés ont la composition molaire suivante :

| | |
|---|---|
| SiO₂/Al₂O₃ | : 40 |
| OH⁻/SiO₂ | : 0,2 |
| (Na+ HM)/Al₂O₃ | : 7 ; HM étant l'hexaméthonium |
| HM/(Na + HM) | : 0,6 |
| H₂O/SiO₂ | : 0,8 |

Les extrudés sont ensuite transférés sur un support en inox localisé dans la partie centrale d'un autoclave en inox de 100 mL (V). Au fond de l'autoclave, on introduit 5 mL d'eau distillée. Le traitement hydrothermal est effectué sans agitation pendant une durée de 1 jour à 180°C (T) par introduction de l'autoclave dans une étuve ventilée Binder de 50 L.
Le produit est récupéré, lavé à l'eau distillée (500 mL) et séché à 100°C. Il est ensuite calciné sous air à 550°C pendant 18 heures. On obtient le matériau M1.
Une analyse par diffraction de rayons X, par comparaison à un échantillon de référence entièrement constitué de zéolithe EU-1 montre que le matériau M1 comprend 5% poids de zéolithe EU-1 correspondant à un taux de conversion de 5% poids. Par une analyse au microscope électronique à balayage, on observe sur la surface externe des extrudés du matériau M1 une dispersion d'agrégats cristallins de zéolithe EU-1 d'une taille de 1 à 5 µm alors que la fracture d'un extrudé révèle un coeur qui présente la même morphologie que l'extrudé de silice initial. Des analyses par microscopie électronique en transmission effectuées sur des échantillons prélevés à la surface et au coeur des extrudés du matériau M1 montrent que la surface est composée de particules cristallines qui font diffracter le faisceau d'électrons alors que l'échantillon prélevé au coeur est amorphe. Le matériau M1 est également analysé par microsonde de Casting : des analyses sont effectuées pas à pas tout au long de la section interne d'un extrudé du matériau M1. Le coefficient de répartition de l'aluminium, noté R(A1), est égal à 1.

### Exemple 2 (invention) : Préparation d'un matériau composite poreux M2 renfermant 15% poids de zéolithe EU-1.

Ce matériau est préparé selon le mode opératoire décrit dans l'exemple 1, mais le traitement hydrothermal est effectué pendant 2 jours à 180°C. A l'issue de la préparation, on obtient le matériau M2. Une analyse par diffraction de rayons X, par comparaison à un échantillon de référence entièrement constitué de zéolithe EU-1 montre que le matériau M2 comprend 15% poids de zéolithe EU-1 correspondant à un taux de conversion de 15% poids. Par une analyse au microscope électronique à balayage on observe sur la surface externe des extrudés et en périphérie sur une épaisseur d'environ 100 µm des agrégats cristallins de zéolithe EU-1 d'une taille de 1 à 10 µm alors que la fracture d'un extrudé révèle un coeur qui présente la même morphologie que l'extrudé de silice initial. Des analyses par microscopie électronique en transmission effectuées sur des échantillons prélevés à la surface et au coeur des extrudés du matériau M2 montrent que la surface est composée de particules cristallines qui font diffracter le faisceau d'électrons alors que l'échantillon prélevé au coeur est amorphe. Le matériau M2 est également analysé par microsonde de Casting : des analyses sont effectuées pas à pas tout au long de la section interne d'un extrudé du matériau M2. Le coefficient de répartition de l'aluminium, noté R(A1), est égal à 1.

### Exemple 3 (invention): Préparation d'un matériau composite poreux M3 renfermant 5% poids de zéolithe EU-1.

Comme structure poreuse et amorphe, on prend 7 g d'extrudés de silice fournie par la société Saint Gobain NorPro, lesquels présentent un diamètre moyen de 1,5 mm, un volume poreux de 1 mL*g⁻¹ et un diamètre moyen des pores de 10 nm.
Ces extrudés de silice sont d'abord imprégnés à sec et à température ambiante avec 6,8 g d'une solution d'hydroxyde d'hexaméthonium à 25% massique préparée selon la procédure décrite à l'exemple 1. Ensuite ces extrudés sont imprégnés à sec et à température ambiante avec une solution comprenant 0,03 g d'aluminate de sodium (Carlo Erba) dans 1 ml d'eau distillée. Les extrudés imprégnés sont ensuite calcinés sous air à 500°C pendant 2 heures.
Les extrudés sont ensuite imprégnés à sec et à température ambiante avec 11 g d'une solution d'hydroxyde d'hexaméthonium à 25% massique. Cette imprégnation est réalisée en 3 étapes. Après chaque imprégnation, les extrudés sont séchés à 80°C pour évaporer l'eau et libérer du volume poreux.
A l'issue de ces étapes d'imprégnation et de séchage, les extrudés ont la composition molaire suivante :

| | |
|---|---|
| SiO₂/Al₂O₃ | : 731 |
| OH- /SiO₂ | : 0,2 |
| (Na + HM)/Al₂O₃ | : 78 ; HM étant l'hexaméthonium |
| HM/(Na + HM) | : 1,0 |
| H₂O/SiO₂ | : 0,8 |

Les extrudés sont ensuite transférés sur un support en inox localisé dans la partie centrale d'un autoclave en inox de 100 mL (V). Au fond de l'autoclave, on introduit 5 mL d'eau distillée. Le traitement hydrothermal est effectué sans agitation pendant une durée de 1 jour à 180°C (T) par introduction de l'autoclave dans une étuve ventilée Binder de 50 L.
Le produit est récupéré, lavé à l'eau distillée (500 mL) et séché à 100°C. Il est ensuite calciné sous air à 550°C pendant 18 heures. On obtient le matériau M3.
Une analyse par diffraction de rayons X, par comparaison à un échantillon de référence entièrement constitué de zéolithe EU-1 montre que le matériau M3 comprend 5% poids de zéolithe EU-1 correspondant à un taux de conversion de 5% poids. Par une analyse au microscope électronique à balayage, on observe sur la surface externe des extrudés dudit matériau M3 une dispersion d'agrégats cristallins de zéolithe EU-1 d'une taille de 1 à 5 µm alors que la fracture d'un extrudé révèle un coeur qui présente la même morphologie que l'extrudé de silice initial. Des analyses par microscopie électronique en transmission effectuées sur des échantillons prélevés à la surface et au coeur des extrudés du matériau M3 montrent que la surface est composée de particules cristallines qui font diffracter le faisceau d'électrons alors que l'échantillon prélevé au coeur est amorphe. Le matériau M3 est également analysé par microsonde de Casting : des analyses sont effectuées pas à pas tout au long de la section interne d'un extrudé du matériau M3. Le coefficient de répartition de l'aluminium, noté R(A1), est égal à 0,2.

### Exemple 4 (invention): Préparation d'un matériau composite poreux M4 renfermant 15% poids de zéolithe EU-1.

Comme structure poreuse et amorphe, on prend 7 g d'extrudés de silice fournie par la société Saint Gobain NorPro, lesquels présentent un diamètre moyen de 1,5 mm, un volume poreux de 1 mL.g⁻¹ et un diamètre moyen des pores de 10 nm.
Ces extrudés de silice sont d'abord imprégnés à sec et à température ambiante avec 6,8 g d'une solution d'hydroxyde d'hexaméthonium à 25% massique préparée selon la procédure décrite à l'exemple 1. Ensuite ces extrudés sont imprégnés à sec et à température ambiante avec une solution comprenant 0,08 g d'aluminate de sodium (Carlo Erba) dans 1 mL d'eau distillée. Les extrudés imprégnés sont ensuite calcinés à 500°C pendant 2 heures.
Les extrudés sont ensuite imprégnés à sec et à température ambiante avec 11 g d'une solution d'hydroxyde d'hexaméthonium à 25% massique. Cette imprégnation est réalisée en 3 étapes. Après chaque imprégnation, les extrudés sont séchés à 80°C pour évaporer l'eau et libérer du volume poreux.
A l'issue de ces étapes d'imprégnation et de séchage, les extrudés ont la composition molaire suivante :

| | |
|---|---|
| SiO₂/Al₂O₃ | : 274 |
| OH⁻/SiO₂ | : 0,2 |
| (Na + HM)/Al₂O₃ | : 30 ; HM étant l'hexaméthonium |
| HM/(Na + HM) | : 0,9 |
| H₂O/SiO₂ | : 0,8 |

Les extrudés sont ensuite transférés sur un support en inox localisé dans la partie centrale d'un autoclave en inox de 100 mL (V). Au fond de l'autoclave, on introduit 5 mL d'eau distillée.
Le traitement hydrothermal est effectué sans agitation pendant une durée de 2 jours à 180°C (T) par introduction de l'autoclave dans une étuve ventilée Binder de 50 L.
Le produit est récupéré, lavé à l'eau distillée (500 mL) et séché à 100°C. Il est ensuite calciné sous air à 550°C pendant 18 heures. On obtient le matériau M4.
Une analyse par diffraction de rayons X, par comparaison à un échantillon de référence entièrement constitué de zéolithe EU-1 montre que le matériau M4 comprend 15% poids de zéolithe EU-1 correspondant à un taux de conversion de 15% poids. Par une analyse au microscope électronique à balayage, on observe sur la surface externe des extrudés et en périphérie sur une épaisseur d'environ 100 µm des agrégats cristallins de zéolithe EU-1 d'une taille de 1 à 10 µm alors que la fracture d'un extrudé révèle un coeur qui présente la même morphologie que l'extrudé de silice initial. Des analyses par microscopie électronique en transmission effectuées sur des échantillons prélevés à la surface et au coeur des extrudés du matériau M4 montrent que la surface est composée de particules cristallines qui font diffracter le faisceau d'électrons alors que l'échantillon prélevé au coeur est amorphe. Le matériau M4 est également analysé par microsonde de Casting : des analyses sont effectuées pas à pas tout au long de la section interne d'un extrudé du matériau M4. Le coefficient de répartition de l'aluminium, noté R(A1), est égal à 0,4.

### Exemple 5 (comparatif) : Préparation d'un matériau composite poreux M5 renfermant 15% poids de zéolithe EU-1.

Ce matériau est préparé selon le mode opératoire décrit dans l'exemple 1, mais le traitement hydrothermal est effectué, sans introduction d'eau distillée au fond de l'autoclave, pendant 3 jours à 180°C. A l'issue de la préparation, on obtient le matériau M5.
Une analyse par diffraction de rayons X, par comparaison à un échantillon de référence montre que le matériau M5 comprend 15% poids de zéolithe EU-1 correspondant à un taux de conversion de 15% poids. Par une analyse au microscope électronique à balayage, on observe à la surface externe ainsi qu'au coeur des extrudés la formation de petites particules cristallines de zéolithe EU-1 d'une taille d'environ 200 nm. Des analyses par microscopie électronique en transmission effectuées sur des échantillons prélevés à la surface et au coeur des extrudés du matériau M5 montrent que les échantillons de surface et de coeur sont composés de particules cristallines qui font diffracter le faisceau d'électrons. Le matériau M5 est également analysé par microsonde de Casting : des analyses sont effectuées pas à pas tout au long de la section interne d'un extrudé. Le coefficient de répartition de l'aluminium, noté R(A1), est égal à 1.

### Exemple 6 (comparatif) : Préparation d'un matériau composite poreux M6 renfermant 15% poids de zéolithe EU-1.

Ce matériau est préparé selon le mode opératoire décrit dans l'exemple 1, mais le traitement hydrothermal est effectué, avec introduction de 0,5 mL d'eau distillée au fond de l'autoclave, pendant 3 jours à 180°C. A l'issue de la préparation, on obtient le matériau M6.
Une analyse par diffraction de rayons X, par comparaison à un échantillon de référence montre que le matériau M6 comprend 15% poids de zéolithe EU-1 correspondant à un taux de conversion de 15% poids. Par une analyse au microscope électronique à balayage, on observe à la surface externe ainsi qu'au coeur des extrudés la formation de petites particules cristallines d'une taille d'environ 200 nm. Des analyses par microscopie électronique en transmission effectuées sur des échantillons prélevés à la surface et au coeur des extrudés du matériau M5 montrent que les échantillons de surface et de coeur sont composés de particules cristallines qui font diffracter le faisceau d'électrons. Le matériau M5 est également analysé par microsonde de Casting : des analyses sont effectuées pas à pas tout au long de la section interne d'un extrudé. Le coefficient de répartition de l'aluminium, noté R(A1), est égal à 1.

### Exemple 7: Préparation des catalyseurs C1 à C6

Les matériaux M1 à M6, calcinés, préparés selon les exemples 1 à 6 sont soumis à 3 échanges ioniques dans une solution de NH₄NO₃ 10N à 100°C pendant 4 heures par échange. Après ces traitements, la zéolithe EU-1 comprise dans ces matériaux calcinés est sous la forme NH₄. Les matériaux M1 à M6 sont imprégnés ensuite à sec avec de l'acide hexachloroplatinique, pour introduire sur chacun des catalyseurs C1 à C6 une teneur massique de platine égale à 0,6%. Les solides sont finalement séchés à 120°C pendant 12 heures et calcinés sous air à 500°C pendant 1 heure. On obtient ainsi les catalyseurs C1, C2, C3, C4, C5 et C6 lesquels sont préparés respectivement à partir des matériaux M1, M2, M3, M4, M5 et M6. Le coefficient de répartition du platine, déterminé par microsonde de Casting, est de 0,4 pour chacun des catalyseurs C1 à C6.

### Exemple 8: Évaluation des propriétés catalytiques des catalyseurs C1 à C6 en isomérisation des aromatiques en C₈.

La charge à isomériser, mise en contact successivement avec chacun des catalyseurs C1 à C6, est constituée uniquement d'éthylbenzène.
Les performances des catalyseurs C1 à C6 issus de l'exemple 7 sont évalués en isomérisation de l'éthylbenzène et en utilisant 5 g de catalyseur.
Les conditions opératoires de l'isomérisation sont les suivantes :
- température : 410°C ;
- pression totale : 10 bar (1 bar = 0,1 MPa);
- pression partielle d'hydrogène : 8 bar.
- charge : éthylbenzène
- vitesse spatiale d'alimentation, exprimée en gramme de charge introduite par gramme de zéolithe présente dans le catalyseur (C1 à C6) et par heure, égale à 66.6 h⁻¹.

Les catalyseurs contenant 15% poids de zéolithe sont testés à une vitesse spatiale, exprimée par gramme de charge introduite par gramme de catalyseur (C2, C4, C5 et C6) et par heure, égale à 10 h⁻¹. Les catalyseurs contenant 5% poids de zéolithe sont testés à une vitesse spatiale, exprimée par gramme de charge introduite par gramme de catalyseur (C1 et C3) et par heure, égale à 3.33 h⁻¹. Les performances des catalyseurs C1 à C6 testés à même débit de charge par quantité de zéolithe sont donc comparables. Les propriétés catalytiques des catalyseurs C1 à C6 sont successivement évaluées pour l'isomérisation de l'éthylbenzène.

Les catalyseurs sont d'abord réduits pendant 2 heures à 400°C sous hydrogène puis ils sont traités avec une charge renfermant du diméthyldisulphide (DMDS) en présence d'hydrogène avec une concentration telle que le rapport atomique soufre/métal est de 1,5. Les catalyseurs sont gardés pendant 3 heures à 400°C dans un courant d'hydrogène, ensuite la charge est injectée.
Les matériaux sont évalués en terme de rendement en isomérisation. Le rendement en xylènes (ou encore rendement en isomérisation) est déterminé à partir des % massiques des xylènes produits, lesdits pourcentages massiques étant obtenus par analyse chromatographique des effluents.

**Tableau 1 : performances des catalyseurs C1 à C6 en terme de rendement en xylènes.**

| catalyseur | C1 | C2 | C3 | C4 | C5 | C6 |
|---|---|---|---|---|---|---|
| | (invention) | (invention) | (invention) | (invention) | (comparatif) | (comparatif) |
| % poids zéolithe EU-1 | 5 | 15 | 5 | 15 | 15 | 15 |
| R(A1) (microsonde de Castaing) | 1 | 1 | 0,2 | 0,4 | 1 | 1 |
| Rdt xylènes (%) | 22,8 | 22,1 | 24,9 | 24,3 | 17,3 | 19,0 |

Les résultats présentés dans le tableau 1 montrent que les catalyseurs C1, C2, C3 et C4 comprenant un matériau composite poreux formé d'un coeur amorphe sur lequel sont dispersés des cristaux de zéolithe EU-1 conduisent à de bien meilleures performances catalytiques en terme de rendement en xylènes que celles obtenues au moyen des catalyseurs C5 et C6 comprenant un matériau composite poreux dont le coeur n'est pas amorphe en raison de la présence de cristaux de zéolithe EU-1.

## Revendications

1. Procédé de préparation d'un matériau composite poreux formé d'un coeur amorphe à base d'un mélange d'oxyde de silicium et d'oxyde d'aluminium sur lequel sont dispersés des cristaux de zéolithe EU-1, ledit procédé comportant au moins les étapes suivantes :
1) imprégnation d'un solide comprenant au moins un oxyde de silicium et au moins un oxyde d'aluminium avec au moins une solution aqueuse comprenant au moins un cation diammonium quaternaire Q de formule [R₁R₂R₃-N-(CH₂)ₙ-N-R₄R₅R₆]²⁺, n étant compris entre 3 et 12, R₁ à R₆, égaux ou différents, sont des groupements alkyles ou hydroxyalkyles avec 1 à 8 atomes de carbone, et jusqu'à 5 des groupements R₁ à R₆ peuvent être des atomes d'hydrogène, chaque étape d'imprégnation selon ladite étape 1) étant suivie d'un séchage à une température inférieure à 200°C,
2) le traitement hydrothermal, mis en oeuvre dans un autoclave de volume V (ml) sous vapeur d'eau et à une température T comprise entre 120 et 220°C, dudit solide issu de l'étape 1), la quantité d'eau préalablement introduite dans ledit autoclave étant strictement supérieure à une quantité volumique égale à V*[23,48*10⁻¹⁰*T³-48*10⁻⁸*T²+5*10⁻⁵*T-0,002] et inférieure ou égale à 0,25*V, et est telle que ledit solide n'est pas en contact direct avec elle,
3) le séchage puis la calcination du solide issu de l'étape 2) de manière à obtenir ledit matériau composite poreux.

2. Procédé de préparation selon la revendication 1 tel que ledit matériau composite poreux se présente sous forme d'extrudés.

3. Procédé de préparation selon la revendication 1 ou la revendication 2 tel que ledit solide comprenant au moins un oxyde de silicium et au moins un oxyde d'aluminium utilisé pour la mise en oeuvre de ladite étape 1) est une silice-alumine amorphe présentant un rapport molaire global SiO₂/Al₂O₃ compris entre 10 et 150.

4. Procédé de préparation selon la revendication 1 ou la revendication 2 tel que ledit solide comprenant au moins un oxyde de silicium et au moins un oxyde d'aluminium utilisé pour la mise en oeuvre de ladite étape 1) est obtenu par imprégnation d'une solution contenant de l'aluminium sur au moins une structure constituée d'oxyde de silicium, le solide issu de l'imprégnation de l'aluminium étant séché à une température inférieure à 200°C et/ou calciné sous air à une température comprise entre 150 et 1000°C.

5. Procédé de préparation selon la revendication 4 tel que ledit solide comprenant au moins un oxyde de silicium et au moins un oxyde d'aluminium utilisé pour la mise en oeuvre de ladite étape 1) présente de l'aluminium réparti de façon homogène au sein de ladite structure constituée d'oxyde de silicium.

6. Procédé de préparation selon la revendication 4 tel que ledit solide comprenant au moins un oxyde de silicium et au moins un oxyde d'aluminium utilisé pour la mise en oeuvre de ladite étape 1) présente de l'aluminium déposé sur la surface externe de ladite structure.

7. Procédé de préparation selon la revendication 6 tel que ledit matériau composite poreux présente un coefficient de répartition de l'aluminium inférieur à 0,5.

8. Procédé de préparation selon l'une des revendications 4 à 7 tel que ledit matériau composite poreux se présente sous la même forme macroscopique que celle de ladite structure constituée d'oxyde de silicium.

9. Procédé de préparation selon l'une des revendications 1 à 8 tel que ledit cation diammonium quaternaire Q présente une formule dans laquelle n est égal à 6 et R₁, R₂, R₃, R₄, R₅ et R₆ sont des groupes méthyles.

10. Procédé de préparation selon l'une des revendications 1 à 9 tel que à l'issue de ladite étape 1), le solide présente la composition molaire suivante :
SiO₂/Al₂O₃ : au moins 10,
OH⁻ /SiO₂ : 0,1 à 6,0,
(M + Q)/Al₂O₃ : 0,5 à 100, M représentant un cation monovalent choisi parmi les métaux alcalins et l'ammonium,
Q/(M + Q) : 0,1 à 1
H₂O/SiO₂ : 0 à 20.

11. Procédé de préparation selon l'une des revendications 1 à 10 tel que ledit traitement hydothermal est réalisé à une température de 180°C dans un autoclave de 100 ml, la quantité d'eau introduite dans le fond dudit autoclave et ne se trouvant pas en contact direct avec le solide étant au moins égale à 4 ml.

12. Procédé de préparation selon l'une des revendications 1 à 11 tel que la synthèse est arrêtée lorsque le taux de conversion en zéolithe EU-1 est inférieur ou égal à 15% poids.

13. Procédé de préparation d'un catalyseur comprenant au moins une étape consistant à imprégner ledit matériau composite poreux préparé selon le procédé selon l'une des revendications 1 à 12 avec au moins un métal du groupe VIII de la classification périodique des éléments.

14. Procédé de préparation d'un catalyseur selon la revendication 13 tel que le(s)dit(s) métal(ux) du groupe VIII est(sont) déposé(s) par imprégnation à sec.

15. Procédé de préparation d'un catalyseur selon la revendication 13 ou la revendication 14 tel qu'il comprend une étape de calcination comprise entre 250°C et 600°C pour une durée comprise entre 0,5 et 10 heures.

16. Procédé d'isomérisation d'une coupe contenant au moins un composé aromatique à huit atomes de carbone par molécule, ledit procédé comprenant la mise en contact de ladite coupe aromatique avec au moins ledit catalyseur préparé selon le procédé selon l'une des revendications 13 à 15 et présent dans un réacteur catalytique.

17. Procédé d'isomérisation selon la revendication 16 tel qu'il est mis en oeuvre dans les conditions opératoires suivantes : une température comprise entre 300°C et 500°C, une pression partielle d'hydrogène comprise entre 0,3 et 1,5 MPa, une pression totale comprise entre 0,45 et 1,9 MPa, une vitesse spatiale d'alimentation, exprimée en kilogramme de charge introduite par kilogramme de catalyseur et par heure, comprise entre 0,25 et 30h⁻¹,

## Patentansprüche

1. Verfahren zur Vorbereitung eines porösen Verbundstoffes, der von einem amorphen Kern auf Basis eines Gemisches von Siliziumoxid und Aluminiumoxid gebildet ist, auf dem Kristalle von Zeolith EU-1 verstreut sind, wobei das Verfahren mindestens die folgenden Schritte umfasst:
1) Imprägnieren eines Feststoffes, umfassend mindestens ein Siliziumoxid und mindestens ein Aluminiumoxid, mit mindestens einer wässrigen Lösung, umfassend mindestens ein quaternäres Diammoniumkation Q der Formel (R₁R₂R₃-N-(CH₂)ₙ-N-R₄R₅R₆]²⁺, wobei n zwischen 3 und 12 beträgt, R₁ bis R₆ gleich oder unterschiedlich und Alkyl- oder Hydroalkylgruppierungen mit 1 bis 8 Kohlenstoffatomen sind, und bis zu 5 Gruppierungen R₁ bis R₆ Wasserstoffatome sein können, wobei auf jeden Imprägnierschritt gemäß Schritt 1) ein Trocknen bei einer Temperatur unter 200 °C erfolgt,
2) hydrothermale Behandlung, die in einem Autoklav mit einem Volumen V (ml) unter Wasserdampf und bei einer Temperatur T zwischen 120 und 200 °C eingesetzt wird, des aus Schritt 1) stammenden Feststoffes, wobei die vorher in den Autoklav eingeleitete Wassermenge exakt größer als eine volumenbezogene Menge gleich V*[23,48*10⁻¹⁰*T³-48*10⁻⁸*T²+5*10⁻⁵*T-0,002] ist und kleiner oder gleich 0,25*V ist, und derart vorgesehen ist, dass der Feststoff mit ihr nicht in direktem Kontakt steht,
3) Trocknen und dann Kalzinieren des aus Schritt 2) stammenden Feststoffes, um den porösen Verbundstoff zu erhalten.

2. Vorbereitungsverfahren nach Anspruch 1, bei dem der poröse Verbundstoff in Form von Extrudat vorhanden ist.

3. Vorbereitungsverfahren nah Anspruch 1 oder Anspruch 2, bei dem der Feststoff, der mindestens ein Siliziumoxid und mindestens ein Aluminiumoxid umfasst, das für den Einsatz des Schrittes 1) verwendet wurde, ein amorphes Siliziumoxid-Alumin ist, das ein globales Molverhältnis SiO₂/Al₂O₃ zwischen 10 und 150 aufweist.

4. Vorbereitungsverfahren nach Anspruch 1 oder Anspruch 2, bei dem der Feststoff, der mindestens ein Siliziumoxid und mindestens ein Aluminiumoxid umfasst, das für den Einsatz des Schrittes 1) verwendet wurde, durch Imprägnieren einer Lösung, die Aluminium auf mindestens einer Struktur, die von Siliziumoxid gebildet ist, umfasst, hergestellt wird, wobei der von dem Imprägnieren des Aluminiums stammende Feststoff bei einer Temperatur unter 200 °C getrocknet und/oder unter Luftabschluss bei einer Temperatur zwischen 150 und 1000 °C kalziniert wird.

5. Vorbereitungsverfahren nach Anspruch 4, bei dem der Feststoff, der mindestens ein Siliziumoxid und mindestens ein Aluminiumoxid umfasst, das für den Einsatz des Schrittes 1) verwendet wurde, Aluminium aufweist, das homogen innerhalb der von Siliziumoxid gebildeten Struktur verteilt ist.

6. Vorbereitungsverfahren nach Anspruch 4, bei dem der Feststoff, der mindestens ein Siliziumoxid und mindestens ein Aluminiumoxid umfasst, das für den Einsatz des Schrittes 1) verwendet wurde, Aluminium aufweist, das auf die Außenfläche der Struktur aufgebracht ist.

7. Vorbereitungsverfahren nach Anspruch 6, bei dem der poröse Verbundstoff einen Verteilungskoeffizienten des Aluminiums unter 0,5 aufweist.

8. Vorbereitungsverfahren nach einem der Ansprüche 4 bis 7, bei dem der poröse Verbundstoff in derselben makroskopischen Form wie jene der von Siliziumoxid gebildeten Struktur vorhanden ist.

9. Vorbereitungsverfahren nach einem der Ansprüche 1 bis 8, bei dem das quaternäre Diammoniumkation Q eine Formel aufweist, in der n gleich 6 ist und R₁, R₂, R₃, R₄, R₅ und R₆ Methylgruppen sind.

10. Vorbereitungsverfahren nach einem der Ansprüche 1 bis 9, bei dem nach dem Schritt 1) der Feststoff die folgende Molzusammensetzung aufweist:
SiO₂/Al₂O₃: mindestens 10
OH-/SiO₂: 0,1 bis 6,0
(M+Q)/Al₂O₃: 0,5 bis 100, wobei M ein monovalentes Kation darstellt, das unter den alkalischen Metallen und Ammonium ausgewählt wird
Q/(M+Q): 0,1 bis 1
H₂O/SiO₂: 0 bis 20.

11. Vorbereitungsverfahren nach einem der Ansprüche 1 bis 10, bei dem die hydrothermale Behandlung bei einer Temperatur von 180 °C in einem Autoklav von 100 ml erfolgt, wobei die in den Boden des Autoklavs eingeleitete und nicht in direktem Kontakt mit dem Feststoff befindliche Wassermenge mindestens gleich 4 ml ist.

12. Vorbereitungsverfahren nach einem der Ansprüche 1 bis 11, bei dem die Synthese angehalten wird, wenn die Konversionsrate in Zeolith EU-1 kleiner oder gleich 15 Gew.-% ist.

13. Verfahren zur Vorbereitung eines Katalysators, umfassend mindestens einen Schritt, der darin besteht, den porösen Verbundstoff, der nach dem Verfahren nach einem der Ansprüche 1 bis 12 vorbereitet wurde, mit mindestens einem Metall der Gruppe VIII des Periodensystems der Element zu imprägnieren.

14. Verfahren zur Vorbereitung eines Katalysators nach Anspruch 13, bei dem das (die) Metall(e) der Gruppe VIII durch Trockenimprägnierung aufgebracht wird (werden).

15. Verfahren zur Vorbereitung eines Katalysators nach Anspruch 13 oder Anspruch 14, das einen Schritt des Kalzinierens zwischen 250 °C und 600 °C für eine Dauer zwischen 0,5 und 10 Stunden umfasst.

16. Verfahren zur Isomerisation eines Schnitts, der mindestens eine aromatische Verbindung mit acht Kohlenstoffatomen pro Molekül enthält, wobei das Verfahren die Herstellung eines Kontakts des aromatischen Schnitts mit mindestens dem Katalysator umfasst, der nach dem Verfahren nach einem der Ansprüche 13 bis 15 vorbereitet wurde und in einem katalytischen Reaktor vorhanden ist.

17. Isomerisationsverfahren nach Anspruch 16, das unter den folgenden Betriebsbedingungen eingesetzt wird: einer Temperatur zwischen 300 °C und 500 °C, einem teilweisen Wasserstoffdruck zwischen 0,3 und 1,5 MPa, einem Gesamtdruck zwischen 0,45 und 1,9 MPa, einer Raumzuleitungsgeschwindigkeit, ausgedrückt in Kilogramm Charge Kilogramm Katalysator und pro Stunde, zwischen 0,25 und 30 h⁻¹.

## Claims

1. Process for the preparation of a porous composite material formed from an amorphous core based on a mixture of silicon oxide and aluminium oxide on which crystals of EU-1 zeolite are dispersed, said process comprising at least the following stages:
1) impregnation of a solid comprising at least one silicon oxide and at least one aluminium oxide with at least one aqueous solution comprising at least one quaternary diammonium cation Q of formula [R₁R₂R₃-N-(CH₂)ₙ-N-R₄R₅R₆]²⁺, n being comprised between 3 and 12, R₁ to R₆, equal or different, are alkyl or hydroxyalkyl groups with 1 to 8 carbon atoms, and up to 5 of the R₁ to R₆ groups can be hydrogen atoms, each stage of impregnation according to said stage 1) is followed by a drying at a temperature below 200°C,
2) the hydrothermal treatment, implemented in an autoclave of volume V (ml) under steam and at a temperature T comprised between 120 and 220°C, of said solid from stage 1), the quantity of water introduced beforehand into said autoclave being strictly greater than a volumetric quantity equal to V*[23.48*10⁻¹⁰*T³-48*10⁻⁸*T²+5*10⁻⁵*T-0.002] and less than or equal to 0.25*V, and is such that said solid is not in direct contact with it,
3) the drying then calcination of the solid from stage 2) so as to obtain said porous composite material.

2. Preparation process according to one of claims 1 to 3 such that said porous composite material is in the form of extrudates.

3. Preparation process according to one of claims 1 or 2 such that said solid comprising at least one silicon oxide and at least one aluminium oxide used for the implementation of said stage 1) is an amorphous silica-alumina having an overall SiO₂/Al₂O₃ molar ratio comprised between 10 and 150.

4. Preparation process according to one of claims 1 or 2 such that said solid comprising at least one silicon oxide and at least one aluminium oxide used for the implementation of said stage 1) is obtained by impregnation of a solution containing aluminium on at least one structure constituted by silicon oxide, said solid obtained from the impregnation of aluminium being dried at a temperature below 200°C and calcined under air at a temperature comprised between 150°C and 1000°C.

5. Preparation process according to claim 4 such that said solid comprising at least one silicon oxide and at least one aluminium oxide used for the implementation of said stage 1) has aluminium distributed homogeneously within said structure constituted by silicon oxide.

6. Preparation process according to claim 4 such that said solid comprising at least one silicon oxide and at least one aluminium oxide used for the implementation of said stage 1) has aluminium deposited on the external surface of said structure.

7. Preparation process according to claim 6 such that said porous composite material has an aluminium distribution coefficient of less than 0.5.

8. Preparation process according to one of claims 4 to 7 such that said porous composite material is in the same macroscopic form as that of said structure constituted by silicon oxide.

9. Preparation process according to one of claims 1 to 10 such that said quaternary diammonium cation Q has a formula in which n is equal to 6 and R₁, R₂, R₃, R₄, R₅ and R₆ are methyl groups.

10. Preparation process according to one of claims 1 to 9 such that at the end of said stage 1), the solid has the following molar composition:
SiO₂/Al₂O₃ : at least 10,
OH⁻/SiO₂ : 0.1 to 6.0,
(M + Q)/Al₂O₃ : 0.5 to 100, M representing a monovalent cation chosen from the alkali metals and ammonium,
Q/(M + Q) : 0.1 to 1
H₂O/SiO₂ : 0 to 20.

11. Preparation process according to one of claims 1 to 10 such that said hydrothermal treatment is carried out at a temperature of 180°C in a 100-ml autoclave, the quantity of water introduced at the bottom of the autoclave and not in direct contact with the solid being at least equal to 4 ml.

12. Preparation process according to one of claims 1 to 11 such that the synthesis is stopped when the rate of conversion to EU-1 zeolite is less than or equal to 15% by weight.

13. Process for the preparation of a catalyst comprising at least one stage involving the impregnation of said porous composite material prepared according to the process according to one of claims 1 to 15 with at least one metal of group VIII of the periodic table of the elements.

14. Process for the preparation of a catalyst according to claim 13 such that said metal(s) of group VIII is(are) deposited by impregnation whilst dry.

15. Process for the preparation of a catalyst according to claim 13 or claim 14 such that it comprises a stage of calcination comprised between 250°C and 600°C for a period comprised between 0.5 and 10 hours.

16. Process of isomerization of a fraction containing at least one aromatic compound with eight carbon atoms per molecule, said process comprising the bringing of said aromatic fraction into contact with at least said catalyst prepared according to the process according to one of claims 16 to 18 and present in a catalytic reactor.

17. Process of isomerization according to claim 16 such that it is implemented under the following operating conditions: a temperature comprised between 300°C and 500°C, a partial hydrogen pressure comprised between 0.3 and 1.5 MPa, a total pressure comprised between 0.45 and 1.9 MPa, a feed space velocity, expressed in kilograms of charge introduced per kilogram of catalyst and per hour, comprised between 0.25 and 30h⁻¹.
